# EUROPÄISCHE PATENTSCHRIFT

(11) **EP 2 943 228 B1**
(45) Veröffentlichungstag und Bekanntmachung des Hinweises auf die Patenterteilung: **08.03.2017**
(21) Anmeldenummer: 14700165.5
(22) Anmeldetag: 08.01.2014
(51) Int. Cl.: A61L 2/22, A61C 19/00, B05B 17/00

(54) **GRUNDKÖRPER UND SYSTEM MIT MEHREREN DÜSEN ZUR REINIGUNG EINES GEGENSTANDS, INSBESONDERE EINES MEDIZINPRODUKTS, SOWIE VERFAHREN ZUM HERSTELLEN EINES SOLCHEN GRUNDKÖRPERS**
MAIN BODY AND SYSTEM COMPRISING MULTIPLE NOZZLES FOR CLEANING AN OBJECT, IN PARTICULAR A MEDICAL PRODUCT, AND METHOD FOR PRODUCING SUCH A MAIN BODY
CORPS DE BASE ET SYSTÈME POURVU DE PLUSIEURS BUSES DE NETTOYAGE D'UN OBJET, EN PARTICULIER D'UN PRODUIT MÉDICAL, ET PROCÉDÉ PERMETTANT DE FABRIQUER UN TEL CORPS DE BASE

(30) Priorität: 08.01.2013 DE 102013000064
(43) Veröffentlichungstag der Anmeldung: 18.11.2015
(73) Patentinhaber: Hysolena GmbH, 6023 Rothenburg (CH)
(72) Erfinder: SAIGER, Lothar, 88525 Dürmentingen (DE)
(74) Vertreter: Kohler Schmid Möbus Patentanwälte
(86) Internationale Anmeldenummer: PCT/EP2014/050231
(87) Internationale Veröffentlichungsnummer: WO 2014/108441

(56) Entgegenhaltungen:
- WO-A1-98/01705
- WO-A1-2011/101214
- WO-A2-2012/101642
- DE-U1-202012 104 279
- US-A1- 2011 139 901

## Beschreibung

Die Erfindung betrifft einen Grundkörper mit mehreren Düsen und ein System mit einem solchen Grundkörper zur Reinigung bzw. Desinfektion eines Gegenstands, insbesondere eines Medizinprodukts, wie beispielsweise eines medizinischen oder zahnmedizinischen Instruments oder eines zahnärztlichen Abdrucks. Die Erfindung betrifft darüber hinaus ein Verfahren zum Herstellen des Grundkörpers.

Medizinprodukte müssen vor ihrer Wiederverwendung am Patienten grundsätzlich eine Hygienekette bestehend aus Reinigung, Desinfektion und ggf. Sterilisation durchlaufen. Dabei sollen möglichst maschinelle Aufbereitungsverfahren eingesetzt werden, um reproduzierbare Reinigungs-, Desinfektions- bzw. Sterilisationsergebnisse zu gewährleisten. In der Praxis ist jedoch bislang kein maschinelles (Reinigungs-) System verfügbar, das für eine effektive Reinigung, Desinfektion und ggf. Sterilisation von Medizinprodukten mit rauer Oberfläche und/oder komplexer Formgebung, wie beispielsweise Knochenraspeln, Fräsen oder zahnärztliche Abdrücken, bzw. auch von Medizinprodukten mit Hohlräumen, beispielsweise Bohrer, Ultraschallspitzen und Ultraschallhandstücke als isolierte Handstücke oder zahnärztliche Handstücke mit festem Schlauch, geeignet ist. Thermolabile Gegenstände wie zahnärztliche Abdrücke sind thermolabil und müssen chemisch desinfiziert werden.

DE 20 2012 104279 U1 offenbart eine Vernebelungsstruktur mit mehreren Düsen zur Abgabe eines Mediums, der scheibenweise aus mehreren hintereinanderliegend angeordneten Platten aufgebaut ist. Der Grundkörper umfasst eine Abdeckplatte sowie eine Verteilerplatte, die der Abdeckplatte fluidisch nachgeschaltet ist und die mit Zuleitungen zum Verteilen des Mediums an die Düsen versehen ist. Die Düsen sind an einer Düsen- oder Vernebelungsplatte angeordnet und zur Stirnseite der Düsenplatte hin offen. Eine als Antriebselement dienende ringförmige Schlussabdeckplatte liegt der Düsenplatte stirnseitig auf.

WO 2012/101642 A2 offenbart eine Zerstäubereinrichtung mit Düsen zum Zerstäuben eines flüssigen Mediums, die aus mehreren aufeinanderliegend angeordneten Platten aufgebaut ist. Eine ähnliche Zerstäubereinrichtung ist aus der WO 2012/101642 A2 bekannt geworden.

Aufgabe der vorliegenden Erfindung ist es, einen Grundkörper mit Düsen zur Abgabe eines Reinigungsmediums oder auch mehrerer Reinigungsmedien, d.h. einen Reinigungskopf, und ein System mit einem solchen Grundkörper zur Reinigung oder Desinfektion eines Gegenstands, insbesondere eines Medizinprodukts wie beispielsweise eines medizinischen und zahnmedizinischen Instruments und eines zahnärztlichen Abdrucks, anzugeben, die eine insgesamt verbesserte Reinigung bzw. Desinfektion auch eines schwer zu reinigenden Gegenstands ermöglichen und die dabei zugleich einen besonders kompakten Aufbau aufweisen. Darüber hinaus soll ein kostengünstiges und wenig aufwendiges Verfahren zur Herstellung des vorgenannten Grundkörpers angegeben werden.

Der Grundkörper weist mehrere Düsen zur Abgabe eines bei der Außenreinigung oder Außendesinfektion des zu reinigenden Gegenstands, insbesondere Medizinprodukts, eingesetzten Reinigungsmediums auf und weist erfindungsgemäß einen scheibenweisen Aufbau mit mehreren hintereinanderliegend angeordneten Platten auf. Der Grundkörper umfasst dabei (einenends) eine Abdeckplatte, die mit einer Zuführleitung für das Reinigungsmedium verbindbar ist. Der Abdeckplatte ist eine Verteilerplatte fluidisch nachgeschaltet, die zum Verteilen des Reinigungsmediums an die Düsen mit Zuleitungen versehen ist. Der Verteilerplatte ist eine Düsenplatte fluidisch nachgeschaltet, die mit den Düsen versehen ist. Die Düsen sind mit den Zuleitungen der Verteilerplatte fluidisch verbunden und zu einer Umfangsseite der Düsenplatte hin offen ausgebildet. Der Grundkörper weist darüber hinaus (anderenends) eine Schlussabdeckplatte auf.

Bei dem erfindungsgemäßen Grundkörper bzw. Reinigungskopf ist die Düsenplatte somit zwischen der Verteilerplatte und der Schlussabdeckplatte angeordnet, sodass das der Düsenplatte über die zugeordnete Verteilerplatte zuströmende Reinigungsmedium über die Düsen der Düsenplatte aus dem Grundkörper herausführbar ist. Durch den erfindungsgemäßen Aufbau des Grundkörpers können die Düsen der Düsenplatte in axialer Richtung des Grundkörpers zu beiden Seiten der Düsenplatte durch die jeweils unmittelbar benachbart angeordnete Platte des Grundkörpers dichtend verschlossen werden. In dem Falle, dass der Grundkörper mit nur einer Düsenplatte versehen ist, ist diese zwischen der Verteilerplatte, die der Düsenplatte fluidisch unmittelbar vorgeschaltet ist, und der Schlußabdeckplatte angeordnet. Die Platten können im Bereich Ihrer Anlageflächen insbesondere (unmittelbar) dichtend aneinander anliegen. Zwischen den einzelnen Platten des Grundkörpers können auch, vorzugsweise ringförmige, Dichtungselemente bzw. Dichtungen angeordnet sein. Die Dichtungselemente/Dichtungen bestehen vorzugsweise aus einem elastomeren, d.h. einem gummi- oder zähelastischen Material. Als zähelastisches Material hat sich insbesondere PTFE bewährt.

In der Verteilerplatte des Grundkörpers sind nach der Erfindung die Zuleitungen zu den Düsen ausgebildet. Die Zuleitungen können erfindungsgemäß so gestaltet sein, dass für jede Düse eine separate Zuleitung besteht, oder aber auch, dass eine Zuleitung jeweils mehrere Düsen miteinander verbindet. Die Zuleitungen können dabei zumindest abschnittsweise als nutartige Vertiefungen der Verteilerplatte ausgebildet sein. Alternativ können die Zuleitungen auch als Durchgangsausnehmungen der Verteilerplatte ausgeführt sein.

In dieser Anmeldung werden unter dem Begriff Reinigungsmedium sowohl Reinigungsmedien wie Wasser, Reiniger oder dergleichen, aber auch Desinfektionslösungen oder Druckluft oder andere Flüssigkeiten oder Gase, beispielsweise zur Gassterilisation durch Ozon oder andere Gase, verstanden.

Der Grundkörper ist somit erfindungsgemäß aus einem Plattenstapel gebildet und kann dadurch besonders kompakt und auf kostengünstige Weise gefertigt werden. Die Düsenplatte kann zwecks einer geringen Verschleißanfälligkeit insbesondere aus einem hochgradig abriebfesten und thermisch belastbaren Material, wie beispielsweise Stahl oder auch einer Stahllegierung, gefertigt sein. Die Düsen können auf einfache Weise aus dem Material der Düsenplatte herausgearbeitet sein. Dies kann insbesondere mittels eines Laserschneidverfahrens, aber auch durch Schleifen, Fräsen, Stanzen oder Bohren erfolgen. Der Grundkörper kann darüber hinaus an unterschiedliche Reinigungsaufgaben, d.h. für die Reinigung eines spezifischen Gegenstands bzw. Medizinprodukts, auf einfache Weise individuell angepasst werden. Der Grundkörper weist erfindungsgemäß eine, vorzugsweise zentrale, Durchgangsausnehmung auf, die sich in Längsrichtung des Grundkörpers, d.h. quer zu den einzelnen Platten des Grundkörpers, durch den Grundkörper hindurcherstreckt. Dadurch kann der Grundkörper den zu reinigenden Gegenstand umgreifend translatorisch entlang des zu reinigenden Gegenstands bewegt werden, um diese vollumfänglich von außen zu behandeln bzw. zu reinigen/zu desinfizieren. Die Düsen des Grundkörpers sind in diesem Falle zur Durchgangsausnehmung des Grundkörpers hin ausgerichtet bzw. offen.

Die Düsen sind nach der Erfindung also randseitig entlang des Innenumfangs der Düsenplatte aufgereiht angeordnet.
Der Grundkörper ist auf die Form/Kontur des zu reinigenden Gegenstands abgestimmt bzw. weist eine der Form/Kontur des zu reinigenden Gegenstands nachempfundene Form und damit Umfangskontur auf.

Dadurch sind die Düsen auf konstruktiv einfachste Weise entlang der der Außenkontur des zu reinigenden Gegenstandes entsprechenden Umfangskontur der Düsenplatte und damit des Grundkörpers angeordnet. Die Düsen der Düsenplatte können darüber hinaus in ihrer räumlichen Anordnung am Grundkörper bzw. in ihrer jeweiligen Spritzcharakteristik auf einfachste Weise auf den zu reinigenden Gegenstand (Medizinprodukt) individuell abgestimmt sein. Dadurch kann der zu reinigende Gegenstand während eines Reinigungsprozesses gezielt mit dem Reinigungsmedium beaufschlagt werden. Die Düsen können dabei die gleiche Spritzcharakteristik aufweisen oder aber sich zumindest teilweise in ihrer jeweiligen Spritzcharakteristik voneinander unterscheiden. Die Düsen können nach der Erfindung insbesondere in ihrem Öffnungsquerschnitt, der Form ihrer Öffnung, ihrem Spritzwinkel (=Öffnungswinkel; Spray vs. Strahl), ihrer Hauptströmungsrichtung (Anstellwinkel der Düse) relativ zur Längsachse des Grundkörpers bzw. des zu reinigenden Gegenstands, und/oder dem Querschnitt und/oder der Länge ihrer jeweiligen Zuleitungen bzw. Zuführungen für das Reinigungsmedium in der Verteilerplatte, gezielt auf den zu reinigenden Gegenstand, d.h. auf dessen Oberflächenkontur (Form) bzw. Oberflächenabschnitte, die unter Reinigungsgesichtspunkten kritisch sind, abgestimmt sein. Dadurch können durch die Düsen jeweils unterschiedliche Reinigungsaufgaben realisiert werden. Insgesamt kann dadurch ein definiertes Reinigungsergebnis besonders effizient, ressourcenschonend und reproduzierbar realisiert werden.

Nach einer bevorzugten Weiterbildung der Erfindung weist der Grundkörper mehrere Düsenplatten auf, denen jeweils eine Verteilerplatte zugeordnet ist. Zwischen zwei Düsenplatten ist dabei jeweils eine Verteilerplatte angeordnet, die der fluidisch unmittelbar nachgeschalteten Düsenplatte zugeordnet ist. Dadurch kann der Grundkörper auf einfache Weise mit mehreren Düsen versehen werden, die in Längsrichtung des Grundkörpers voneinander beabstandet angeordnet sind. Die Reinigung bzw. Desinfektion des Gegenstands kann dadurch nochmals weiter verbessert und beschleunigt werden. Es versteht sich, dass die Verteiler- und Düsenplatten in diesem Fall zumindest teilweise mit Durchführungsausnehmungen versehen sind, über die das Reinigungsmedium jeweils der fluidisch nachgeschalteten Verteilerplatte zuführbar ist.

Für das Zuführen unterschiedlicher Reinigungsmedien an die Düsenplatten weist der Grundkörper im konstruktiv einfachsten Fall eine Trennplatte auf, die zwischen einer ersten und einer zweiten Düsenplatte angeordnet ist. Die Trennplatte ist dabei der Verteilerplatte der zweiten Düsenplatte fluidisch vorgeschaltet. Es versteht sich, dass die Abdeckplatte in diesem Falle mit getrennten Durchführungen für die Reinigungsmedien und die Verteilerplatte der ersten Düsenplatte, die erste Düsenplatte und auch die Trennplatte jeweils mit einer Durchführung für das für die zweite Düsenplatte bestimmte Reinigungsmedium versehen sind.

Nach der Erfindung kann/können auch die Abdeckplatte und/oder die Schlussabdeckplatte mit Düsen versehen sein. Die Zuführung des Reinigungsmediums an die Düsen der Abdeckplatte erfolgt über die Verteilerplatte, die der Abdeckplatte unmittelbar fluidisch nachgeschaltet angeordnet ist. Die Zuführung des Reinigungsmediums an die Düsen der Schlussabdeckplatte erfolgt im Hinblick auf einen kompakten und einfachen Aufbau des Grundkörpers vorteilhaft über die der Schlussabdeckplatte fluidisch unmittelbar vorgeschaltete Düsenplatte des Grundkörpers.

Die Platten des Grundkörpers können insbesondere miteinander verschraubt, verhakt oder miteinander verschweißt, verlötet oder verklebt sein. Die Platten können im erstgenannten Fall vorteilhaft jeweils mit zumindest zwei Durchgangsbohrungen für Verbindungsmittel versehen sein, wobei die Durchgangsbohrungen der Platten in axialer Richtung miteinander fluchten. Die eingesetzten Verbindungsmittel erstrecken sich durch die Durchgangsbohrungen der einzelnen Platten hindurch, um die Platten miteinander zu verbinden bzw. gegeneinander zu verspannen. In den drei letztgenannten Fällen können sich zusätzliche Dichtungselemente zwischen den einzelnen Platten erübrigen.

Zur nochmals verbesserten Reinigung des Gegenstands bzw. Medizinprodukts können an dem Grundkörper ein oder mehrere zusätzliche Reinigungselemente, beispielsweise Bürsten oder Reinigungstücher, angebracht sein. Der Grundkörper kann nach der Erfindung einen Turbinenrotor aufweisen, mittels dessen die zusätzlichen Reinigungselemente um eine Rotationsachse relativ zum Grundkörper bewegbar sind. Der Turbinenrotor ist im konstruktiv einfachsten Fall durch das dem Grundkörper zugeführte Reinigungsmedium antreibbar. Ein Volumenstromanteil des dem Grundkörper zugeführten Reinigungsmediums wird dazu dem Turbinenrotor zugeführt.

Die Abdeckplatte des Grundkörpers ist vorzugsweise derart ausgestaltet, dass an dieser eine Kolbenstange montierbar ist, mittels derer der Grundkörper hin-und herbewegbar ist und über die das Reinigungsmedium dem Grundkörper zuführbar ist. Dadurch kann der Grundkörper auch bei einem (Reinigungs-)System, d.h. bei einem Reinigungsautomaten, eingesetzt werden. Mittels der Kolbenstange kann der Grundkörper im Reinigungs- bzw. Desinfektionsbetrieb bzw. im Bearbeitungsbetrieb relativ zu den zu reinigenden bzw. zu bearbeitenden Gegenständen bewegt werden.

Zum Aufheizen des Reinigungsmediums bzw. der Reinigungsmedien kann der Grundkörper erfindungsgemäß ein Heizelement aufweisen. Das Heizelement kann entweder durch eine oder mehrere Platten des Grundkörpers selbst gebildet sein oder aber als ein zu den Platten des Grundkörpers separates Bauteil ausgebildet sein. Im erstgenannten Fall ist/sind die Platte(n) als ein Widerstandsheizelement ausgebildet.

Eine elektrische Anschlussleitung des vorstehend genannten Heizelements kann dem Grundkörper beispielsweise über die vorstehend genannte Kolbenstange zugeführt sein. Die Kolbenstange kann alternativ oder zusätzlich mit einer Heizung zum Aufheizen des Reinigungsmediums/der Reinigungsmedien versehen sein.

Der Grundkörper kann nach der Erfindung darüber hinaus als eine Lichtabgabeeinheit ausgebildet sein, um Licht mit einer definierten Wellenlänge bzw. einem definierten Wellenlängenspektrum an einen zu behandelnden, insbesondere zu reinigenden bzw. zu desinfizierenden oder auch zu sterilisierenden Gegenstand abzugeben. Das Licht ist dabei vorzugsweise von außen, beispielsweise über einen Lichtleiter, in den Grundkörper einkoppelbar. Der Grundkörper kann zumindest teilweise aus einem lichtleitenden Material bestehen. Darüber hinaus kann der Grundkörper mit einem Umlenkmittel, beispielsweise einem Spiegel, versehen sein, mittels dessen der Lichtstrahl gezielt auf den zu behandelnden Gegenstand gelenkt werden kann. Zur Desinfektion des Gegenstands kann insbesondere UV-Licht eingesetzt werden.

Das Verfahren zur Herstellung eines vorgenannten Grundkörpers weist die folgenden Verfahrensschritte auf:
Zunächst werden die Düsen in die Düsenplatte eingebracht, derart, das die Düsen entlang des Innenumfangs der Düsenplatte hintereinander aufgereiht angeordnet und zur Durchgangsausnehmung hin offen sind. In einem weiteren Schritt werden die Zuleitungen in der Verteilerplatte eingebracht.

Die Düsen und/oder die Zuleitungen können aus der jeweiligen Platte mit einem Laser herausgeschnitten oder aber herausgebohrt, gefräst oder geschliffen werden.

In einem weiteren Schritt wird die Abdeckplatte und die Schlussabdeckplatte bereitgestellt.

In einem weiteren Schritt werden die Abdeckplatte, die Verteilerplatte, die Düsenplatte und die Schlussabdeckplatte aufeinandergelegt bzw. aufeinandergestapelt und unter Bildung des Grundkörpers miteinander verbunden. Dies kann durch Verschweißen, Verkleben, Verlöten oder auch durch Verschrauben der vorgenannten Platten erfolgen.

Das erfindungsgemäße Verfahren erlaubt eine besonders einfache und kostengünstige Herstellung eines Grundkörpers mit mehreren Düsen, die zumindest teilweise eine gemeinsame Zuführung für das/die Reinigungsmedien oder auch jeweils getrennte Zuführung aufweisen können.

Zwischen den einzelnen Platten können erfindungsgemäß Dichtungselemente angeordnet werden, durch die die Platten gegeneinander und in radialer Richtung flüssigkeitsdicht gegeneinander abgedichtet werden. Die Dichtungselemente können dabei insbesondere lose zwischen den Platten positioniert und durch das nachfolgende gemeinsame Verbinden der Platten zwischen diesen im Klemmsitz gehalten angeordnet werden.

Die Platten können für einen besonders sicheren Dichtsitz der Dichtungselemente an ihren einander zuweisenden Seitenflächen mit einer Dichtungsaufnahmestruktur, insbesondere einer Ringnut, oder dergl., versehen werden, in die die Dichtungselemente eingelegt werden.

Das erfindungsgemäße System zur Reinigung eines Gegenstands, insbesondere eines Medizinprodukts, weist in an sich bekannter Weise eine Aufbereitungskammer auf, in der der zu reinigende Gegenstand bzw. das Medizinprodukt angeordnet ist. Das System ist mit zumindest einem vorgenannten Grundkörper versehen, wobei der Grundkörper an einer Kolbenstange oder an einem anderen Linearantrieb montiert ist, sodass der Grundkörper mittels der Kolbenstange bzw. mittels des Linearantriebs in der Aufbereitungskammer zum Reinigen des Gegenstands bzw. Medizinprodukts relativ zu dem Gegenstand hin- und herbewegbar ist.

Der Grundkörper ist somit mit einer Kolbenstange verbunden, in der bzw. an der sich vorzugsweise die Verrohrung befindet, durch die das Reinigungsmedium bzw. die Reinigungsmedien dem Grundkörper und somit den Düsen zuführbar ist. Verschiedene Reinigungsmedien können dabei durch eine Zuleitung oder auch durch verschiedene Zuleitungen zum Grundkörper transportiert werden.

Die Kolbenstange kann Bestandteil eines Kolbens sein, der die Kolbenstange so bewegt, dass der Grundkörper mit den Düsen an dem zu reinigenden Gegenstand entlang geführt wird und so den Gegenstand in seiner gesamten Länge reinigt und/oder chemisch desinfiziert. Der Kolben ist vorzugsweise elektrisch oder pneumatisch betrieben. Anstelle eines Kolbens kann auch ein Zylinder eingesetzt werden.

Die Aufbereitungskammer des (Reinigungs-)Systems kann zwecks einer vereinfachten Handhabung des Systems einen für das Reinigungsmedium durchlässigen Behälter aufweisen, in den der zu reinigende Gegenstand bzw. die zu reinigenden Gegenstände/Medizinprodukte einlegbar sind. Der Behälter kann im konstruktiv einfachsten Fall als eine Gitterbox ausgebildet sein. Der Behälter kann der Aufbereitungskammer vorzugsweise entnehmbar sein. Dadurch kann die Aufbereitungskammer vereinfacht mit dem zu reinigenden Gegenstand beschickt bzw. dieser nach einem erfolgten Reinigungsprozess vereinfacht der Aufbereitungskammer entnommen werden.

Der Behälter weist bevorzugt Halterungen für den zu reinigenden Gegenstand bzw. das zu reinigende Medizinprodukt auf, die auf den zu reinigenden Gegenstand bzw. das zu reinigende Medizinprodukt individuell abgestimmt sind. Dadurch kann sichergestellt werden, dass der zu reinigende Gegenstand in einer auf eine optimale Reinigung der Gegenstände ausgerichteten (vorgegebenen) Position und Lage relativ zum Grundkörper, d.h. zum Reinigungskopf, angeordnet ist.

Die Aufbereitungskammer kann erfindungsgemäß einen Adapter zum Anschluss eines als Hohlinstrument ausgebildeten Gegenstands (Medizinprodukts) aufweisen. Dadurch kann das Reinigungsmedium auch durch das Innere des zu reinigenden Gegenstands/Medizinprodukts geführt werden.

Das System weist bevorzugt mehrere der vorstehend erläuterten Grundkörper mit Düsen auf. Dadurch kann die zeitgleiche bzw. zeitversetzte Reinigung mehrerer und/oder unterschiedlicher Medizinprodukte bzw. Gegenstände realisiert werden. Dies ist für die Effizienz und Einsatzflexibilität des Systems von Vorteil. Die Kolbenstangen der Grundkörper sind dabei vorzugsweise voneinander beabstandet und zueinander parallel verlaufend angeordnet, sodass eine gegenseitige Behinderung der Bewegung der Grundkörper innerhalb der Aufbereitungskammer sicher ausgeschlossen werden kann. Darüber hinaus kann dadurch einer gegenseitigen Beeinträchtigung des Reinigungs- bzw. Desinfektionsvermögens der einzelnen Grundkörper zuverlässig entgegengewirkt und unerwünschte Spülschatten vermieden werden. Die Grundkörper weisen vorzugsweise einen gemeinsamen Antrieb auf.

Das System weist anschlussseitig bevorzugt einen Ventilblock und einen Verteilerblock zum Verteilen des Reinigungsmediums oder der Reinigungsmedien an den Grundkörper und/oder den Adapter auf.
Nach einer bevorzugten Weiterbildung der Erfindung ist/sind in den Verteilerblock Bauteile zur Kühlung und/oder eine interne Heizung und/oder eine externe Heizung und/oder eine Dosiereinheit für Prozessmittel und/oder Komponenten zur Prozessüberwachung eingebaut.

Nachfolgend wird die Erfindung anhand eines in der Zeichnung wiedergegebenen Ausführungsbeispiels näher erläutert.

In der Zeichnung zeigen:
- Fig. 1:: ein erfindungsgemäßes System zur Außenreinigung bzw. Außendesinfektion eines Gegenstands, hier eines Medizinprodukts, mit einer Aufnahmekammer für den zu reinigenden Gegenstand und mit einem in der Aufnahmekammer bewegbar angeordneten Grundkörper mit Düsen zur Abgabe eines Reinigungsmediums, wobei der Grundkörper scheibenweise aus einer Mehrzahl von aufeinandergestapelten Einzelplatten aufgebaut ist und die Düsen an einem Randabschnitt in einer als Düsenplatte ausgebildeten Einzelplatte des Grundkörpers ausgebildet ist, in einer Draufsicht;
- Fig. 2:: die Abdeckplatte des Grundkörpers aus Fig. 1 mit einer Durchführung für das Reinigungsmedium, in einer Draufsicht;
- Fig. 3:: die Düsenplatte des Grundkörpers aus Fig. 1 mit den innenumfangsseitig angeordneten Düsen, in einer Draufsicht;
- Fig. 4:: die Verteilerplatte des Grundkörpers aus Fig. 1 mit in der Verteilerplatte ausgebildeten Zuleitungen zu den Düsen der in Fig. 3 gezeigten Düsenplatte, in einer Draufsicht;
- Fig. 5:: eine alternative Ausführungsform des Grundkörpers aus Fig. 1, bei dem der Grundkörper mit zusätzlichen Reinigungselementen versehen ist, in Seitenansicht,
- Fig. 6:: eine alternative Ausführungsform des Grundkörpers aus Fig. 1 mit mehreren Düsen- und Verteilerplattenpaaren;
- Fig. 7:: eine alternative Ausführungsform des Grundkörpers aus Fig. 1 mit mehreren Düsenplatten, denen unterschiedliche Reinigungsmedien zuführbar sind, in einer Seitenansicht; und
- Fig. 8:: ein Blockdiagramm mit einzelnen Verfahrensschritten des erfindungsgemäßen Verfahrens zum Herstellen des in Fig. 1 gezeigten Grundkörpers.

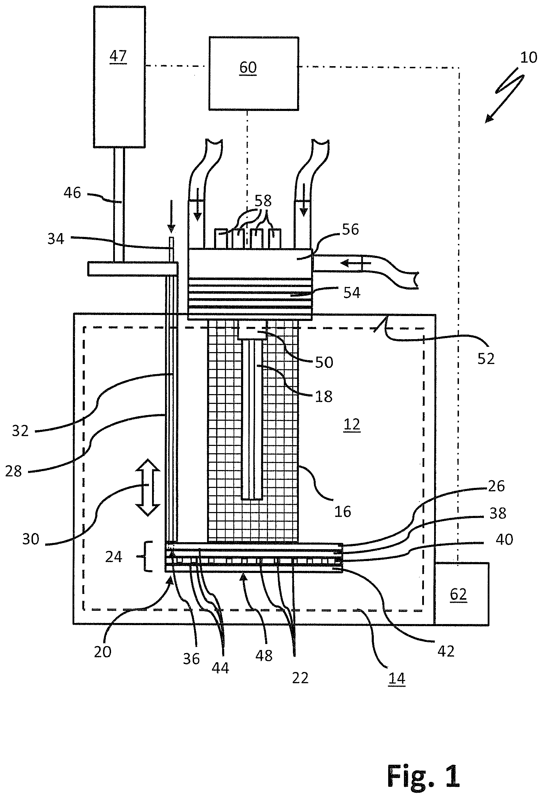

**Fig. 1** zeigt ein System **10** zur Reinigung eines Gegenstands, insbesondere Medizinprodukts, wie beispielsweise eines medizinischen/zahnärztlichen Instruments, in einer schematischen Darstellung. Das System 10 weist eine Aufbereitungskammer **12** mit einem öffenbaren Deckel **14** auf. In der Aufbereitungskammer 12 ist ein als Gitterkorb ausgebildeter Behälter **16** angeordnet, in den ein zu reinigender Gegenstand **18**, hier ein zahnärztliches Instrument, angeordnet ist. Der Behälter **16** ist für ein Reinigungsmedium derart durchlässig, dass der Gegenstand 18 durch diesen hindurch von außen gereinigt werden kann.

Bei einer anderen Ausführungsform wird der zu reinigende Gegenstand **18** in eine spezielle Halterung in den Behälter 16 eingelegt. So kann eine besonders intensive Reinigung des Gegenstands 18 gewährleistet werden, ohne dass dieser unter dem Druck des Reinigungsmediums ausweichen kann.

Zur Außenreinigung des Gegenstands 18 dient ein Grundkörper **20**, d.h. ein Reinigungskopf, der mehrere Düsen **22** zur gezielten Abgabe eines Reinigungsmediums an den Gegenstand 18 aufweist. Der Grundkörper 20 ist bei dem in Fig. 1 gezeigten Ausführungsbeispiel ringförmig ausgebildet und scheibenweise aus mehreren Platten aufgebaut. Der Grundkörper 20 umfasst mit anderen Worten einen Plattenstapel **24**. Eine mit **26** bezeichnete Abschlussplatte des Grundkörpers 20 dient der Befestigung des Grundkörpers 20 an einer Kolbenstange **28**, die in Pfeilrichtung **30** hin- und herbewegbar ist. Die Kolbenstange erstreckt sich durch die Wandung der Aufbereitungskammer 12 hindurch nach außen.

Die Kolbenstange 28 weist in ihrem Inneren eine Leitung **32** auf, über die das Reinigungsmedium dem Grundkörper 20 zuführbar ist. An die Leitung 32 ist einlassseitig eine Zuführleitung **34**, hier ein Schlauch, für das beim Reinigungsprozess eingesetzte Reinigungsmedium angeschlossen.

Die Abschlussplatte 26 weist eine Durchführung **36** für das Reinigungsmedium auf. An die Abschlussplatte 26 grenzt eine Verteilerplatte **38** an, die der Verteilung des Reinigungsmediums an die Düsen 22 einer Düsenplatte 40 dient. An die Düsenplatte grenzt auf der von der Abschlussplatte 26 abgewandten Seite der Düsenplatte 40 eine Schlussabdeckplatte **42** an.

Die Form der Düsen 22, die Dicke der Platten, der Querschnitt der Zuleitung zu den Düsen 22 und die Anzahl der Düsen 22 beeinflussen den Druck, mit dem das Reinigungsmedium auf den zu reinigenden Gegenstand 18 auftrifft und damit die Reinigungsleistung. Der Aufbau der Düsen 22 entscheidet darüber, ob das Reinigungsmedium als Strahl oder als Spray aus der Düse 22 austritt.

Die vorstehend genannten Platten des Grundkörpers 20 sind in nicht näher wiedergegebener Weise miteinander verschraubt, können aber auch miteinander verhakt, verschweißt, verlötet oder verklebt oder in anderer Weise dichtend miteinander verbunden sein. Zwischen den einzelnen Platten kann, wie dies in Fig. 1 gezeigt ist, jeweils ein Dichtungselement **44** aus einem elastomeren Material angeordnet sein, um die einzelnen Platten des Grundkörpers 20 gegeneinander flüssigkeitsdicht abzudichten.

Die Kolbenstange ist mit einer außerhalb der Aufbereitungskammer angeordneten weiteren Kolbenstange **46** bewegungsgekoppelt verstellbar. Die weitere Kolbenstange ist in einem Pneumatik-Zylinder **47** geführt. Nach einem in der Zeichnung nicht näher wiedergegebenen alternativen Ausführungsbeispiel kann die Kolbenstange 28 auch elektrisch, hydraulisch oder magnetisch antreibbar sein.

Der Grundkörper 20 weist im vorliegenden Beispiel eine auf das zu reinigende Medizinprodukt 18 abgestimmte (Grund-)Form mit einer Durchgangsausnehmung **48** auf, die größer ist, als der Behälter 16. Dadurch kann der Grundkörper 20 bei einer Betätigung der Kolbenstangen 28, 46 den Behälter 16 umgreifend relativ zu dem zu reinigenden Gegenstand über den Behälter 16 sowie den darin angeordneten zu reinigenden Gegenstand hinwegbewegt werden. Bei einer Bewegung des Grundkörpers relativ zu dem zu reinigenden Gegenstand 18 erstreckt sich der Gegenstand 18 somit durch die Durchgangsausnehmung 48 des Grundkörpers hindurch. Die Durchgangsausnehmung 48 des Grundkörpers 20 ist durch einzelne Durchgangsausnehmungen der Platten des Grundkörpers 20 gebildet. Die Düsen 22 des Grundkörpers 20 sind innenumfangsseitig am Grundkörper 20 angeordnet und zur Durchgangsausnehmung 48 des Grundkörpers 20 hin offen.

Das vorliegende System ist darüber hinaus auch zur Innenreinigung von Hohlinstrumenten, wie beispielsweise Ultraschallhandstücken oder zahnärztlichen Turbinenhandstücken oder dergleichen, ausgelegt.

Die Aufbereitungskammer 12 weist dazu einen Adapter **50** zum Anschluss des innenseitig zu reinigenden bzw. zu desinfizierenden Gegenstands 18 auf. Der Adapter 50 ist vorliegend an einer Innenwand **52** der Aufbereitungskammer 12 angeordnet, kann aber grundsätzlich auch bodenseitig in der Aufbereitungskammer 12 angeordnet sein. Bei zahnärztlichen Übertragungsinstrumenten mit festem Schlauch wird beispielsweise die Zuleitung, durch die Wasser, beispielsweise an die Ultraschallspitze gedrückt wird, mit dem Adapter 50 verbunden. Vorliegend ist der als Hohlinstrument ausgebildete Gegenstand 18 an dem Adapter 650 angeschlossen.

Wie aus Fig. 1 weiter hervorgeht, weist das System 10 einen Verteilerblock **54** mit Ventilblock **56** auf, mittels derer ein oder mehrere Reinigungsmedien zeitgleich oder zeitversetzt dem Grundkörper 20 mit den Düsen 22 bzw. dem Adapter 50 zur Innenreinigung des zu reinigenden Gegenstandes zuführbar ist. Durch das Öffnen eines oder mehrerer Ventile 58 strömt das Reinigungsmedium durch den Ventilblock 56 hindurch in den Verteilerblock 54 der das Reinigungsmedium dann gezielt zur Außenreinigung des Gegenstands 18 an den Grundkörper 20 bzw. den Adapter 50 zur Innenreinigung des als Hohlkörper ausgebildeten Gegenstands 18 verbringt. Wasser, Reiniger, Desinfektionsmittel, Druckluft oder Gase können so durch die Zuleitung bzw. den Gegenstand 18 hindurch gepresst werden und diese reinigen und desinfizieren.

Bei einer thermischen Desinfektion bzw. Sterilisation des Gegenstands 18 innerhalb der Aufbereitungskammer 12 kann das Restwasser, das nach der Reinigung in der Zuleitung verbleibt, verdampfen. Des Weiteren kann man in die heiße Zuleitung Wasser einbringen, das dann augenblicklich verdampft. Der so entstehende Wasserdampf kann dabei zur Heißreinigung eingesetzt werden. Diese Methode erlaubt auch die Aufbereitung von Endoskopen.

Eine mit **60** bezeichnete Steuereinheit dient dem Steuern aller Betriebs- bzw. Prozessparameter des Systems. Eine Anzeige- und Bedieneinheit **62** des Systems dient als Benutzerschnittstelle und ist mit der Steuereinheit 60 verbunden.

Es versteht sich, dass das System 10 auch mehrere der vorstehend erläuterten Grundkörper 20 aufweisen kann. Die Grundkörper 20 können sich dabei in Formgebung und Aufbau voneinander unterscheiden, um unterschiedlich ausgeformte Gegenstände 18 zu behandeln.

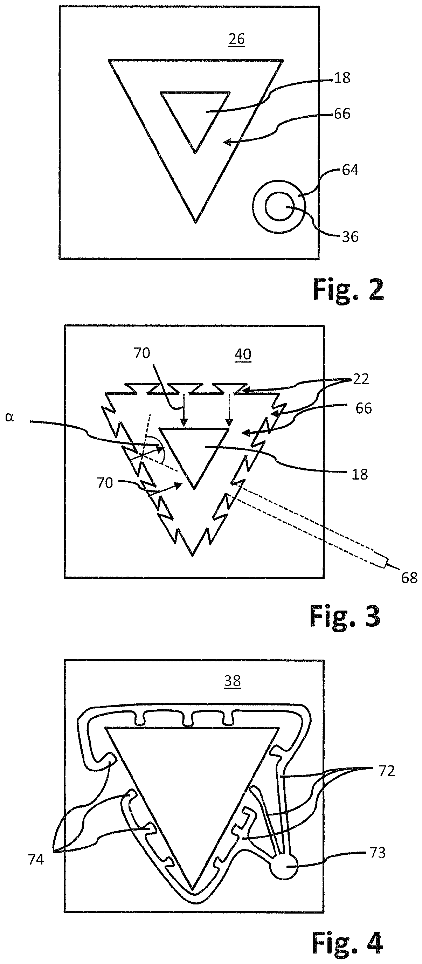

**Fig. 2** zeigt die Abschlussplatte 26 des in Fig. 1 gezeigten Grundkörpers 20 mit der Durchführung 36 für das über die Kolbenstange heranströmende Reinigungsmittel in einer Draufsicht. Die Abschlussplatte 26 weist einen Befestigungsflansch **64** für die Kolbenstange (Fig. 1) auf. Eine zentrale Durchgangsausnehmung der Abdeckplatte 26 ist mit **66** bezeichnet. Die Durchgangsausnehmung 66 der Abdeckplatte 26 weist eine zur Umfangskontur (Form) des zu reinigenden Gegenstands 18 korrespondierende auf Form auf.

In **Fig. 3** ist eine weitere Einzelplatte des in Fig. 1 gezeigten Grundkörpers 20, hier die Düsenplatte 40 in einer beispielhaften Ausgestaltung gezeigt. Die Düsenplatte 40 weist eine zentrale Durchgangsausnehmung **66** auf, die der zentralen Durchgangsausnehmung der in Fig. 2 gezeigten Abdeckplatte entspricht. Die Düsen 22 sind entlang des Innenumfangs der Düsenplatte 40 hintereinander aufgereiht angeordnet und sind zur Durchgangsausnehmung 66 der Düsenplatte 40 hin offen.

Die Düsen 22 weisen in dem hier gezeigten Ausführungsbeispiel eine einheitliche Formgebung mit einem einheitlichen Düsenwinkel α auf. Die Düsen 22 sind mit einem Laser aus der Düsenplatte heraus geschnitten, d.h. freigeschnitten (eingearbeitet) worden.

Der mögliche Durchfluss und der Druck des Reinigungsmediums werden durch den Öffnungsquerschnitt **68** der Düsen beeinflusst. Eine jeweilige Hauptstömungsachse **70** der Düsen 22 ist mit 68 bezeichnet. Die Hauptströmungsachse 70 der Düsen entspricht einem jeweiligen Anstellwinkel (Ausrichtung) der Düsen 22 bezogen auf die Längsachse des Grundkörpers.

Die Position und Lage der Düsen 22 an der Düsenplatte 40 sowie deren jeweilige weiteren Düsenparameter, d.h. deren Öffnungsquerschnitt 68, Hauptströmungsrichtung 68 sowie deren Zuleitungen bzw. Zuführungen (Zuführkanäle) für das Reinigungsmedium in der Verteilerplatte sind gezielt auf den zu reinigenden Gegenstand, d.h. auf dessen Oberflächenkontur und unter Reinigungsgesichtspunkten kritische Oberflächenabschnitte ausgerichtet.

**Fig. 4** zeigt die Verteilerplatte 38 des in Fig. 1 gezeigten Grundkörpers 20 in einer freigestellten Draufsicht. Die Zuleitungen **72** zu den Düsen der in der in Fig. 2 wiedergegebenen Düsenplatte 40 sind gut zu erkennen.

Die Zuleitungen 72 können als Vertiefungen oder als Durchgangsausnehmungen der Verteilerplatte 38 ausgebildet sein. Die Zuleitungen sind allesamt über eine Durchgangsbohrung **73** mit der Durchführung der Abschlussplatte (Fig. 2) fluidisch verbunden. Die Zuleitungen 72 können aus der, beispielsweise aus Edelstahl gefertigten, Düsenplatte, herausgelasert, -gebohrt, -gefräst oder -geschliffen sein. Natürlich sind auch andere Plattenmaterialien denkbar. Das Material der Verteilerplatte 38 hängt untere anderem von dem bzw. den (Reinigungs-) Medium/Medien ab, die durch die Verteilerplatte 38 geleitet werden.

Die Kanäle bzw. Zuleitungen 72 mehrerer Düsen 22 können zusammengefasst sein. Wie in Fig. 4 gezeigt ist, kann auch eine Zuleitung 72 einer einzelnen Düse individuell zugeordnet sein. Die knopfartigen Ausbuchtungen **74** der Zuführungen stellen die fluidische Verbindung der Zuführungen bzw. Zuleitungen 72 zu den einzelnen Düsen der in Fig. 2 gezeigten Düsenplatte her. Diese knopfartigen Ausbuchtungen 74 sind als Durchgangsbohrungen der Verteilerplatte 38 ausgebildet und münden in den der Düse 22 fluidisch vorgeschalteten Hohlraum (nicht bezeichnet) der Düsenplatte.

Der jeweilige Durchfluss und der Druck des Reinigungsmediums an der einzelnen Düse hängt unter anderem auch vom Querschnitt der Zuführung, der Länge der Zuführung und der Reinigungsdüse selbst ab. Besondere Formen im End- bzw. Austrittsbereich der Düsen 22 können zu gewünschten Verwirbelungen des Reinigungsmediums und/oder zur Spraybildung führen.

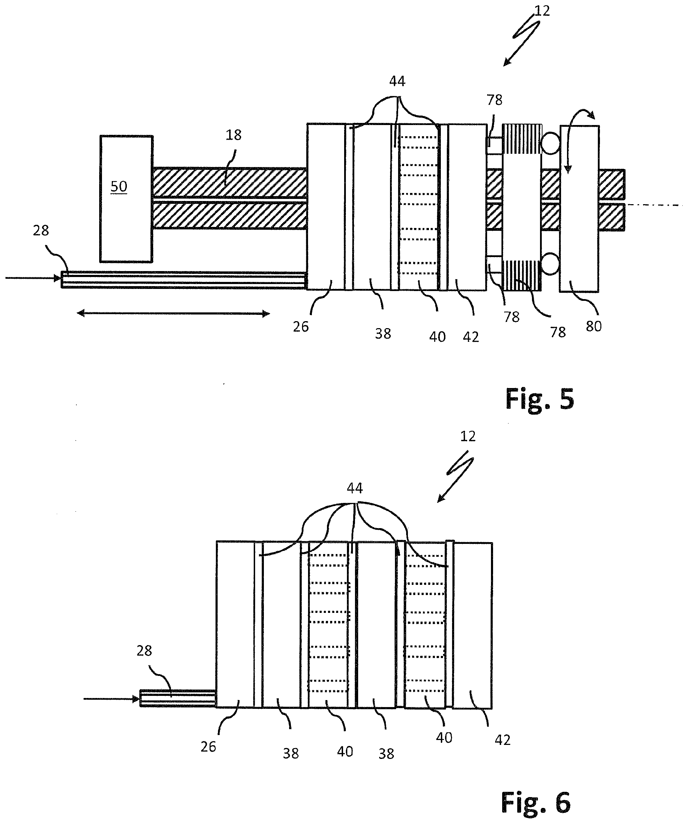

**Fig. 5** zeigt eine weitere Ausführungsform des Grundkörpers 20 mit einer Abschlussplatte 26, einer Verteilerplatte 38, einer Düsenplatte 40 und einer Schlussabdeckplatte 42.

Die Dichtungselemente 44 bzw. Dichtungen zwischen den einzelnen Platten sind gut zu erkennen. An der Abschlussplatte 26 ist die Kolbenstange 28 befestigt.

Der Grundkörper unterscheidet sich von dem im Zusammenhang mit Fig. 1 erläuterten Grundkörper darin, dass dieser eine Befestigungsmöglichkeit **76**, d.h. Befestigungsmittel, für ein erstes (zusätzliches) Reinigungselement **78** aufweist. Das Reinigungselement 78 kann, wie dies in Fig. 5 beispielhaft gezeigt ist, als Bürste oder auch in Form von Reinigungstüchern (nicht gezeigt) ausgebildet sein.

An dem Reinigungselement 78 kann über eine weitere Befestigung darüber hinaus ein (weiteres) und als Turbinenrotor **80** ausgebildetes Reinigungselement angebracht sein, das -relativ zum Grundkörper 20 -an diesem beweglich gelagert ist. Beispielsweise kann die bewegliche Lagerung des am Grundkörper 20 über ein Kugellager realisiert sein. Es versteht sich, dass auch das Reinigungselement 78 am Grundkörper 20 beweglich angebracht bzw. gelagert und ggf. als Turbinenrotor ausgebildet sein kann.

Gemäß einer in der Zeichnung nicht näher gezeigten Ausführungsform des Grundkörpers 20 kann dieser einen Aufsatz aufweisen, der so gestaltet ist, dass damit das zu reinigende Instrument durch die Bewegung des Grundkörpers 20 gedreht werden kann.

In **Fig. 6** ist ein weiterer Grundkörper gezeigt, der sich von dem in Fig. 1 gezeigten Grundkörper im Wesentlichen darin unterscheidet, dass dieser zwei paarweise angeordnete Verteilerplatten-Düsenplatten-Einheiten aufweist, die in Reihe geschaltet sind.

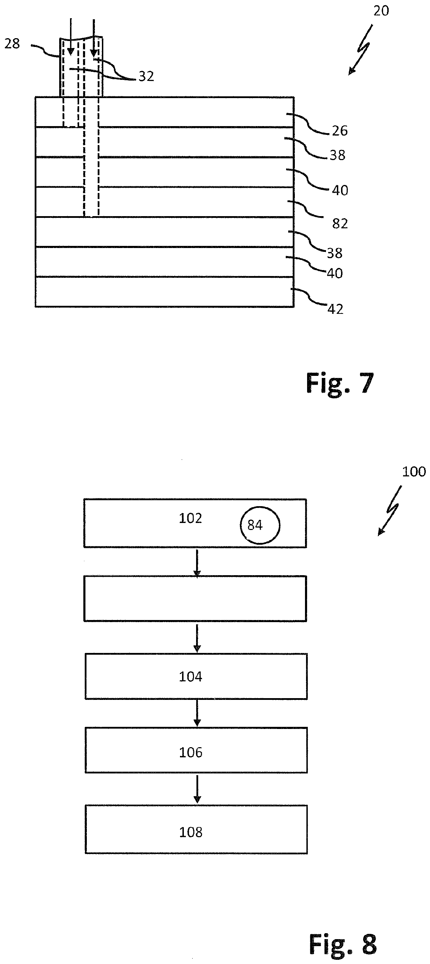

**Fig. 7** zeigt den prinzipiellen Aufbau eines Grundkörpers 20 mit Düsen, bei dem zwei unterschiedliche Reinigungsmedien eingesetzt werden können.

Der Grundkörper 20 weist zwei paarweise hintereinanderliegend angeordnete Verteiler- und Düsenplatten 38, 40 auf, die durch eine einzige Trennplatte **82** voneinander getrennt sind. Der Grundkörper 20 ist einenends durch die Abdeckplatte 26 und anderenends durch die Schlussabdeckplatte 42 begrenzt.

Der Grundkörper 20 ist mit zwei getrennten Zuführleitungen 32 für die beiden Reinigungsmedien fluidisch verbunden. Die in der Figur 7 links dargestellte Zuführleitung 32 ist über eine erste Durchführung (nicht gezeigt) der Abdeckplatte 26 mit der in der Figur oberen (ersten) Verteilerplatte 38 der in der Figur oberen (ersten) Düsenplatte und über die Zuführungen (nicht gezeigt) dieser Verteilerplatte 38 mit den Düsen (nicht gezeigt) der ersten Düsenplatte 40 fluidisch verbunden.

Die in der Figur 7 rechts dargestellte zweite Zuführleitung ist über eine zweite Durchführung (nicht gezeigt) der Abdeckplatte und Durchgangsbohrungen der Verteilerplatten 38 sowie der ersten Düsenplatte 40 und der Trennplatte 82 mit den Düsen der in der Figur unteren (zweiten) Düsenplatte fluidisch verbunden.

Die Düsen der beiden Düsenplatten 40 sind am Grundkörper 20 derart angeordnet, dass sich die Reinigungsmedien der beiden Düsenplatten 40 nach dem Verlassen der Düsen zumindest teilweise miteinander vermischen. Das Mischungsverhältnis wird durch den gewählten Durchfluss des Reinigungsmediums durch die jeweiligen Düsen und die gemeinsame Überdeckung der jeweiligen Medienstrahlen beeinflusst. Zur Bildung eines Mediensprays kann eines der beiden Reinigungsmedien gasförmig, beispielsweise Druckluft, sein.

**Fig. 8** zeigt ein Blockdiagramm mit einzelnen Verfahrensschritten des erfindungsgemäßen Verfahrens **100** zum Herstellen des im Zusammenhang mit den Figuren 1 bis 3 erläuterten Grundkörpers.

In Schritt 102 werden die Düsen 22 in die Düsenplatte 40 eingebracht. Mit anderen Worten werden die Düsen 22 in eine Edelstahlplatte bzw. einen anderen Düsenplattenrohling unter Bildung der Düsenplatte eingebracht.

In einem weiteren Schritt **104** werden die Zuleitungen in die Verteilerplatte eingebracht. Mit anderen Worten werden die Zuleitungen in eine Edelstahlplatte bzw. einen anderen Verteilerplattenrohling unter Bildung der Verteilerplatte eingebracht.

Die Düsen 22 und/oder die Zuleitungen 72 können aus der jeweiligen Platte jeweils mit einem Laser herausgeschnitten oder aber herausgebohrt, gefräst oder geschliffen werden.

In einem weiteren Schritt **106** werden die Abdeckplatte 26 und die Schlussabdeckplatte 42 bereitgestellt.

In einem abschließenden Schritt **108** werden die Abdeckplatte 26, die Verteilerplatte 38, die Düsenplatte 40 und die Schlussabdeckplatte 42 aufeinandergelegt bzw. aufeinandergestapelt und unter Bildung des Grundkörpers 20 miteinander verbunden.

Die Formgebung der einzelnen Platten wird derart gewählt, dass der zu bildende Grundkörper mit seiner im Betrieb dem zu reinigenden Gegenstand zugewandten Umfangsfläche (=Oberfläche) an die (Grund-)form bzw. die Grundkontur des zu reinigenden Gegenstands 18 (Fig. 1) angepasst ist. Mit anderen Worten weisen die Platten, insbesondere die Düsenplatte, zumindest abschnittsweise eine auf die Grundform bzw. die Grundkontur des zu reinigenden Gegenstands abgestimmte, insbesondere eine dieser entsprechende, Umfangskontur auf. Die Anordnung der Düsen entlang der Umfangskontur der Düsenplatte und damit des Grundkörpers sowie die Spritzeigenschaften der einzelnen Düsen werden individuell auf die Grundform des zu reinigenden Gegenstands abgestimmt.

Bei dem Verfahrensschritt 108 können zwischen den einzelnen Platten Dichtungselemente 44 angeordnet werden, um einen unerwünschten Austritt der im Betriebseinsatz in den Grundkörper strömenden Reinigungsmediums außerhalb der dafür vorgesehenen Düsen 22 zu unterbinden.

Mit dem beschriebenen Verfahren kann der Grundkörper 20 ganz individuell für die jeweilige Reinigungs- bzw. Desinfektionsaufgabe, d. h. spezifisch für den zu reinigenden Gegenstand, mit Düsen inklusive deren Zuleitungen und Anschlüssen herstellt werden.

## Patentansprüche

1. Grundkörper (20) zur Reinigung oder Desinfektion eines Gegenstands (18), insbesondere eines Medizinprodukts, wie beispielsweise eines medizinischen oder zahnärztlichen Instruments oder eines zahnärztlichen Abdrucks, aufweisend mehrere Düsen (22) zur Abgabe eines Reinigungsmediums,
wobei der Grundkörper (20) scheibenweise aus mehreren hintereinanderliegend angeordneten Platten (26, 38, 40, 42) aufgebaut ist, umfassend:
a. eine Abdeckplatte (26), die mit einer Zuführleitung (34) für ein Reinigungsmedium verbindbar ist,
b. eine Verteilerplatte (38), die der Abdeckplatte (26) fluidisch nachgeschaltet ist und die mit Zuleitungen (72) zum Verteilen des Reinigungsmediums an die Düsen (22) versehen ist,
c. eine Düsenplatte (40), die mit den Düsen (22) versehen ist, wobei die Düsen (22) mit den Zuleitungen (72) der Verteilerplatte (40) fluidisch verbunden und zu einer Seite der Düsenplatte (40) hin offen sind, und
d. eine Schlussabdeckplatte (42),
**dadurch gekennzeichnet, dass** die Abdeckplatte (26) und die Düsenplatte (40) jeweils eine zentrale Durchgangsausnehmung (66) aufweisen, die eine zur Umfangskontur des zu reinigenden Gegenstands (18) korrespondierende Form aufweist, wobei die Düsen (22) entlang des Innenumfangs der Düsenplatte (40) hintereinander aufgereiht angeordnet und zur Durchgangsausnehmung (66) hin offen sind.

2. Grundkörper nach Anspruch 1, **dadurch gekennzeichnet, dass** die Düsen (22) zumindest teilweise voneinander unterschiedlich beabstandet an der Düsenplatte (40) angeordnet sind.

3. Grundkörper nach Anspruch 1 oder 2, **dadurch gekennzeichnet, dass** zwischen den einzelnen Platten (26, 38, 40, 42) des Grundkörpers (20), vorzugsweise ringförmige, Dichtungselemente (44) angeordnet sind.

4. Grundkörper nach einem der vorhergehenden Ansprüche, **dadurch gekennzeichnet, dass** die Platten (26, 38, 40, 42) des Grundkörpers miteinander verschraubt, verschweißt, verlötet oder miteinander verhakt sind.

5. Grundkörper nach einem der vorhergehenden Ansprüche, **dadurch gekennzeichnet, dass** an dem Grundkörper (22) ein zusätzliches Reinigungselement (78), beispielsweise eine Bürste oder ein Reinigungstuch, angeordnet ist.

6. System (10) zur Reinigung oder Desinfektion eines Gegenstands (18), insbesondere eines Medizinprodukts, umfassend eine Aufbereitungskammer (12), in der der zu reinigende Gegenstand (18) angeordnet ist, und einen Grundkörper (20) mit mehreren Düsen (22) zur Abgabe eines Reinigungsmediums,
**dadurch gekennzeichnet, dass**
der Grundkörper nach einem der Ansprüche 1 bis 5 ausgebildet ist, wobei der Grundkörper (20) in seiner Form an die Form des zu reinigenden Gegenstands (18) angepasst ist und wobei der Grundkörper (20) in der Aufbereitungskammer (12) an dem zu reinigenden Gegenstand (18) entlangführbar ist.

7. System nach Anspruch 6, **dadurch gekennzeichnet, dass** die Düsen (22) in ihrer Spritzcharakteristik, also in ihrem Öffnungsquerschnitt (68), ihrem Spritzwinkel (α) und/oder ihrer Hauptstömungsrichtung (70), jeweils auf den zu reinigenden Gegenstand (18) spezifisch abgestimmt sind.

8. System nach Anspruch 6 oder 7, **dadurch gekennzeichnet, dass** der Grundkörper an einer Kolbenstange (28) montiert ist, mittels derer der Grundkörper (20) an dem zu reinigenden Gegenstand (18) entlangführbar ist.

9. System nach Anspruch 8, **dadurch gekennzeichnet, dass** die Kolbenstange (28) eine Leitung (32) aufweist, über die das Reinigungsmedium dem Grundkörper (20) zuführbar ist.

10. System nach Anspruch 8 oder 9, **dadurch gekennzeichnet, dass** die Kolbenstange (28) pneumatisch oder elektrisch antreibbar ist.

11. System nach einem der Ansprüche 6 bis 10, **dadurch gekennzeichnet, dass** die Aufbereitungskammer (12) einen für das Reinigungsmedium durchlässigen Behälter (16) aufweist, in den der zu reinigende Gegenstand (18) eingelegt ist, und/oder dass die Aufbereitungskammer (12) einen Adapter (50) zum Anschluss eines als Hohlinstrument ausgebildeten Gegenstands (18) aufweist, über den das Reinigungsmedium dem Gegenstand (18) zur Innenreinigung des Gegenstands (18) zuführbar ist.

12. System nach einem der vorhergehenden Ansprüche 6 bis 11, **gekennzeichnet durch** zwei oder mehr Grundkörper (20), die in der Aufbereitungskammer (12) bewegbar angeordnet sind, wobei die Grundkörper (20) bevorzugt mit einem gemeinsamen Antrieb versehen sind.

13. Verfahren (100) zum Herstellen eines Grundkörpers (20) nach einem der vorhergehenden Ansprüche 1 bis 5, **gekennzeichnet durch** die folgenden Schritte:
- Einbringen (102) der Düsen (22) in die Düsenplatte (40), derart, dass die die Düsen (22) entlang des Innenumfangs der Düsenplatte (40) hintereinander aufgereiht angeordnet und zur Durchgangsausnehmung (66) hin offen sind;
- Einbringen (104) der Zuleitungen (72) in die Verteilerplatte (38);
- Bereitstellen (106) der Abdeckplatte (26) und der Schlussabdeckplatte (42); und
- Aufeinanderstapeln und miteinander Verbinden (108) der Abdeckplatte (26) der Verteilerplatte (38), der Düsenplatte (40) und der Schlussabdeckplatte (42) unter Bildung des Grundkörpers.

14. Verfahren nach Anspruch 13, **dadurch gekennzeichnet, dass** die Düsen (22) in der Düsenplatte (40) und/oder die Zuleitungen (72) in der Verteilerplatte (38) herausgelasert werden oder dass die Düsen (22) aus der Düsenplatte (40) und/oder die Zuleitungen (72) aus der Verteilerplatte (38) gebohrt, gefräst, gestanzt oder herausgeschliffen werden.

15. Verfahren nach Anspruch 13 oder 14, **dadurch gekennzeichnet, dass** zwischen den einzelnen Platten (26, 38, 40, 42) Dichtungselemente (44) angeordnet werden.

## Claims

1. Basic body (20) for cleaning or disinfecting an article (18), in particular a medical device, for example a medical or dental instrument or a dental impression, having a plurality of nozzles (22) for dispensing a cleaning medium,
wherein the basic body (20) is constructed in layers from a plurality of plates (26, 38, 40, 42) arranged one after another, comprising:
a. a cover plate (26) which is connectable to a feed line (34) for a cleaning medium,
b. a distributor plate (38) which is fluidically connected downstream of the cover plate (26) and which is provided with feed lines (72) for distributing the cleaning medium to the nozzles (22),
c. a nozzle plate (40) which is provided with the nozzles (22), wherein the nozzles (22) are fluidically connected to the feed lines (72) of the distributor plate (40) and are open towards one side of the nozzle plate (40), and
d. a terminating cover plate (42),
**characterized in that** the cover plate (26) and the nozzle plate (40) each have a central through-aperture (66) which has a shape corresponding to the peripheral contour of the article (18) to be cleaned, wherein the nozzles (22) are arranged one after another in a row around the inner periphery of the nozzle plate (40) and are open towards the through-aperture (66).

2. Basic body according to Claim 1, **characterized in that** the nozzles (22) are arranged on the nozzle plate (40) at spacings from one another that are different at least in part.

3. Basic body according to Claim 1 or 2, **characterized in that** preferably annular seal elements (44) are arranged between the individual plates (26, 38, 40, 42) of the basic body (20).

4. Basic body according to one of the preceding claims, **characterized in that** the plates (26, 38, 40, 42) of the basic body are screwed, welded or soldered to or interlocked with one another.

5. Basic body according to one of the preceding claims, **characterized in that** an additional cleaning element (78), for example a brush or a cleaning cloth, is arranged on the basic body (22).

6. System (10) for cleaning or disinfecting an article (18), in particular a medical device, comprising a treatment chamber (12) in which the article (18) to be cleaned is arranged, and a basic body (20) having a plurality of nozzles (22) for dispensing a cleaning medium,
**characterized in that**
the basic body is configured according to one of Claims 1 to 5, wherein the shape of the basic body (20) is matched to the shape of the article (18) to be cleaned and wherein the basic body (20) is able to be guided in the treatment chamber (12) along the article (28) to be cleaned.

7. System according to Claim 6, **characterized in that** the nozzles (22) are each specifically coordinated with the article (18) to be cleaned in terms of their spray characteristics, i.e. in terms of their opening cross section (68), their spray angle (α) and/or their main direction of flow (70).

8. System according to Claim 6 or 7, **characterized in that** the basic body is mounted on a piston rod (28), by means of which the basic body (20) is able to be guided along the article (18) to be cleaned.

9. System according to Claim 8, **characterized in that** the piston rod (28) has a conduit (32), via which the cleaning medium is able to be fed to the basic body (20).

10. System according to Claim 8 or 9, **characterized in that** the piston rod (28) is pneumatically or electrically drivable.

11. System according to one of Claims 6 to 10, **characterized in that** the treatment chamber (12) has a container (16) which is permeable to the cleaning medium and into which the article (18) to be cleaned is introduced, and/or **in that** the treatment chamber (12) has an adapter (50) for the attachment of an article (18) in the form of a hollow instrument, via which the cleaning medium is able to be fed to the article (18) in order to internally clean the article (18).

12. System according to one of the preceding Claims 6 to 11, **characterized by** two or more basic bodies (20) which are arranged in a movable manner in the treatment chamber (12), wherein the basic bodies (20) are preferably provided with a common drive.

13. Method (100) for producing a basic body (20) according to one of the preceding Claims 1 to 5, **characterized by** the following steps of:
- introducing (102) the nozzles (22) into the nozzle plate (40) such that the nozzles (22) are arranged one after another in a row around the inner periphery of the nozzle plate (40) and are open towards the through-aperture (66);
- introducing (104) the feed lines (72) into the distributor plate (38);
- providing (106) the cover plate (26) and the terminating cover plate (42); and
- stacking and connecting together (108) the cover plate (26), the distributor plate (38), the nozzle plate (40) and the terminating cover plate (42), forming the basic body.

14. Method according to Claim 13, **characterized in that** the nozzles (22) in the nozzle plate (40) and/or the feed lines (72) in the distributor plate (38) are lasered out, or **in that** the nozzles (22) are drilled, milled, punched or ground out of the nozzle plate (40) and/or the feed lines (72) are drilled, milled, punched or ground out of the distributor plate (38).

15. Method according to Claim 13 or 14, **characterized in that** seal elements (44) are arranged between the individual plates (26, 38, 40, 42).

## Revendications

1. Corps de base (20) pour le nettoyage ou la désinfection d'un objet (18), en particulier d'un produit médical, par exemple un instrument médical ou dentaire ou une empreinte dentaire, présentant plusieurs buses (22) pour délivrer un agent de nettoyage,
le corps de base (20) étant construit par tranches constituées de plusieurs plaques disposées les unes derrière les autres (26, 38, 40, 42),
comprenant :
a. une plaque de recouvrement (26) qui peut être raccordée à une conduite d'alimentation (34) pour un agent de nettoyage,
b. une plaque de distribution (38) qui est montée fluidiquement après la plaque de recouvrement (26) et qui est pourvue de conduites d'amenée (72) pour distribuer l'agent de nettoyage aux buses (22),
c. une plaque à buses (40) qui est pourvue des buses (22), les buses (22) étant reliées fluidiquement aux conduites d'amenée (72) de la plaque de distribution (40) et étant ouvertes vers un côté de la plaque à buses (40), et
d. une plaque de recouvrement terminale (42),
**caractérisé en ce que** la plaque de recouvrement (26) et la plaque à buses (40) présentent chacune un évidement de passage central (66) qui présente une forme correspondant au contour périphérique de l'objet à nettoyer (18), les buses (22) étant disposées le long de la périphérie intérieure de la plaque à buses (40) en rangées les unes derrière les autres et étant ouvertes vers l'évidement de passage (66).

2. Corps de base selon la revendication 1, **caractérisé en ce que** les buses (22) sont disposées au moins en partie à des distances différentes les unes des autres sur la plaque à buses (40).

3. Corps de base selon la revendication 1 ou 2, **caractérisé en ce qu'**entre les plaques individuelles (26, 38, 40, 42) du corps de base (20) sont disposés des éléments d'étanchéité (44) de préférence annulaires.

4. Corps de base selon l'une quelconque des revendications précédentes, **caractérisé en ce que** les plaques (26, 38, 40, 42) du corps de base sont vissées, soudées, brasées les unes aux autres ou accrochées les unes aux autres.

5. Corps de base selon l'une quelconque des revendications précédentes, **caractérisé en ce qu'**un élément de nettoyage supplémentaire (78), par exemple une brosse ou une étoffe de nettoyage, est disposé sur le corps de base (22).

6. Système (10) pour le nettoyage ou la désinfection d'un objet (18), en particulier d'un produit médical, comprenant une chambre de préparation (12) dans laquelle est disposé l'objet à nettoyer (18), et un corps de base (20) comprenant plusieurs buses (22) pour distribuer un agent de nettoyage,
**caractérisé en ce que**
le corps de base est réalisé selon l'une quelconque des revendications 1 à 5, la forme du corps de base (20) étant adaptée à la forme de l'objet à nettoyer (18) et le corps de base (20) pouvant être guidé dans la chambre de préparation (12) le long de l'objet à nettoyer (18).

7. Système selon la revendication 6, **caractérisé en ce que** les buses (22), par leurs caractéristiques de pulvérisation, c'est-à-dire leur section transversale d'ouverture (68), leur angle de pulvérisation (α) et/ou leur direction d'écoulement principale (70), sont spécifiquement adaptées à chaque fois à l'objet à nettoyer (18).

8. Système selon la revendication 6 ou 7, **caractérisé en ce que** le corps de base est monté sur une tige de piston (28) au moyen de laquelle le corps de base (20) peut être guidé le long de l'objet à nettoyer (18).

9. Système selon la revendication 8, **caractérisé en ce que** la tige de piston (28) présente une conduite (32) par le biais de laquelle l'agent de nettoyage peut être acheminé au corps de base (20).

10. Système selon la revendication 8 ou 9, **caractérisé en ce que** la tige de piston (28) peut être entraînée pneumatiquement ou électriquement.

11. Système selon l'une quelconque des revendications 6 à 10, **caractérisé en ce que** la chambre de préparation (12) présente un récipient (16) perméable pour l'agent de nettoyage, dans lequel est introduit l'objet à nettoyer (18), et/ou **en ce que** la chambre de préparation (12) présente un adaptateur (50) pour le raccordement d'un objet (18) réalisé sous forme d'instrument creux, par le biais duquel l'agent de nettoyage peut être acheminé à l'objet (18) pour le nettoyage intérieur de l'objet (18).

12. Système selon l'une quelconque des revendications 6 à 11, **caractérisé par** deux ou plus de deux corps de base (20) qui sont disposés de manière déplaçable dans la chambre de préparation (12), les corps de base (20) étant pourvus de préférence d'un entraînement commun.

13. Procédé (100) de fabrication d'un corps de base (20) selon l'une quelconque des revendications précédentes 1 à 5, **caractérisé par** les étapes suivantes :
- introduction (102) des buses (22) dans la plaque à buses (40), de telle sorte que les buses (22) soient disposées le long de la périphérie intérieure de la plaque à buses (40) en rangées les unes derrière les autres et soient ouvertes vers l'évidement de passage (66) ;
- introduction (104) des conduites d'amenée (72) dans la plaque de distribution (38) ;
- fourniture (106) de la plaque de recouvrement (26) et de la plaque de recouvrement terminale (42) ; et
- empilement et assemblage les unes aux autres (108) de la plaque de recouvrement (26), de la plaque de distribution (38), de la plaque à buses (40) et de la plaque de recouvrement terminale (42) en formant le corps de base.

14. Procédé selon la revendication 13, **caractérisé en ce que** les buses (22) sont pratiquées par découpage au laser dans la plaque à buses (40) et/ou les conduites d'amenée (72) sont réalisées par découpage au laser dans la plaque de distribution (38) ou **en ce que** les buses (22) sont percées, fraisées, estampées ou meulées dans la plaque à buses (40) et/ou les conduites d'amenée (72) sont percées, fraisées, estampées ou meulées dans la plaque de distribution (38).

15. Procédé selon la revendication 13 ou 14, **caractérisé en ce qu'**entre les plaques individuelles (26, 38, 40, 42) sont disposés des éléments d'étanchéité (44).
